# EUROPÄISCHE PATENTSCHRIFT

(11) **EP 2 982 741 B1**
(45) Veröffentlichungstag und Bekanntmachung des Hinweises auf die Patenterteilung: **10.01.2018**
(21) Anmeldenummer: 15179257.9
(22) Anmeldetag: 31.07.2015
(51) Int. Cl.: C12M 1/107, C12M 1/00, C12M 1/34, F17B 1/24, G01F 22/02

(54) **BIOMASSESPEICHER**
BIOMASS STORAGE
ACCUMULATEUR A BIOMASSE

(30) Priorität: 08.08.2014 DE 202014103688 U
(43) Veröffentlichungstag der Anmeldung: 10.02.2016
(73) Patentinhaber: JOPE Beteiligungs GmbH, 87653 Eggenthal (DE)
(72) Erfinder:
(74) Vertreter: Pfister & Pfister Patent- und Rechtsanwälte

(56) Entgegenhaltungen:
- EP-A2- 2 078 937
- DE-A1-102012 112 935
- DE-U1-202008 003 091
- DE-U1-202012 104 130

## Beschreibung

Die Erfindung betrifft einen Biomassespeicher zum Vergären von Biomasse. Insbesondere, aber nicht ausschließlich betrifft die Erfindung einen Biomassespeicher nach dem Oberbegriff von Anspruch 1.

Biomassespeicher zum Speichern und Vergären von Biomasse sind allgemein bekannt. Ein solcher Biomassespeicher umfasst ein im Wesentlichen starres Behälterelement zur Aufnahme der Biomasse. Das Behälterelement ist mit einer Abdeckhaube abgedeckt. Unterhalb der Abdeckhaube ist eine Gasspeicherfolie angeordnet, die zum Verschließen des Behälterelements dient und die an einem Rand des Behälterelements befestigt ist, so dass der Biomassespeicher im Wesentlichen luftdicht abgeschlossen ist. Ein solcher Biomassespeicher ist in DE 10 2012 112935 A1 offenbart. Durch das Vergären der Biomasse entstehen Gase, die sich unter der Gasspeicherfolie sammeln. Diese Gase sind der wirtschaftliche Sinn und Zweck für das Betreiben einer Biogasanlage, da sie zur Energiegewinnung genutzt oder gewinnbringend verkauft werden können. Für einen wirtschaftlichen Betrieb eines Biomassespeichers ist es wichtig, den Vergärungsprozess möglichst lange Zeit am Stück und ohne Unterbrechungen laufen zu lassen, um eine maximal mögliche Biogasmenge pro Zeit zu erzielen. Allerdings ist bei der beschriebenen Art von Biomassespeichern die Gefahr gegeben, dass durch einen nicht überwachten Gärungsprozess so viel Biogas erzeugt wird, dass die Gasspeicherfolie zu stark gespannt wird und somit reißt oder anderweitig beschädigt wird. Derartige Beschädigungen wiederum führen zu längeren Ausfällen der Anlage. Für einen optimalen und sicheren Betrieb müssen Biomassespeicher ohne Unterbrechung des Gärungsprozesses soviel Biogas wie möglich erzeugen, allerdings ohne dass die Anlage zu stark belastet und beschädigt wird. In der Praxis erweist sich ein derartiger, optimaler Betrieb eines herkömmlichen Biomassespeichers allerdings als schwierig, da die Gaserzeugung durch den Vergärungsprozess im Allgemeinen nicht gleichmäßig und konstant über die Zeit erfolgt und so Wartungs- und Wechselintervalle für die Biomassespeicher kaum theoretisch berechnet werden können.
Aufgabe der vorliegenden Erfindung ist es daher eine Möglichkeit zu schaffen, die in einem Biomassespeicher befindliche Gasmenge zuverlässig und kontinuierlich zu bestimmen.
Die Aufgabe wird gelöst durch einen Biomassespeicher zum Vergären von Biomasse, der ein im Wesentlichen starres Behälterelement zur Aufnahme der Biomasse und eine Abdeckhaube und eine unterhalb der Abdeckhaube angeordnete Gasspeicherfolie zum Verschließen des Behälterelements umfasst, die an einem Rand des Behälterelements befestigt ist, so dass der Biomassespeicher im Wesentlichen luftdicht abgeschlossen ist und eine Gasvolumenerfassungsvorrichtung zum Ermitteln und Anzeigen eines von der Gasspeicherfolie überspannten Gasvolumens in dem Biomassespeicher, wobei an der Gasspeicherfolie mindestens ein Versteifungselement angeordnet ist. Ein erfindungsgemäßer Biomassenspeicher umfasst ein im Wesentlichen starres Behälterelement mit einer Abdeckhaube. Das starre Behälterelement kann beispielsweise aus Betonringen aufgebaut sein. Oberhalb des im Wesentlichen starren Behälterelementes ist eine Abdeckhaube vorgesehen, die dazu dient, Witterungseinflüsse von dem Biomassespeicher abzuhalten. Die Abdeckhaube kann dabei beispielsweise als gewebeverstärkte Plane, als über ein Gestell gespannte Plane oder als Traglufthaube ausgebildet sein. Ein im Wesentlichen luftdichter oder gasdichter Verschluss des starren Behälterelementes nach oben erfolgt durch eine Gasspeicherfolie, die sich unterhalb der schützenden Abdeckhaube befindet. Die Gasspeicherfolie ist am Rand des im Wesentlichen starren Behälterelementes angebracht, wobei der vom starren Behälterelement und der Gasspeicherfolie begrenzte Raum gegenüber der Umgebung abgedichtet ist. Die zu vergärende Biomasse befindet sich innerhalb dieses nach außen abgedichteten Raumes. Die bei der Vergärung anfallenden Gase verbleiben ebenfalls innerhalb dieses, durch das starre Behälterelement und die Gasspeicherfolie begrenzten Raumes. Je länger der Vergärungsprozess abläuft, desto mehr Gas wird erzeugt. Die erzeugte Gasmenge benötigt mit der Zeit daher immer mehr Volumen in dem Biomassespeicher. Das führt dazu, dass sich die Gasspeicherfolie nach oben wölbt um der vergrößerten Gasmenge Raum zu verschaffen. Der Erfinder hat erkannt, dass eine Bestimmung der im Biomassespeicher befindlichen Gasmenge über die Wölbung bzw. die Form der Gasspeicherfolie mit Hilfe einer Gasvolumenerfassungsvorrichtung bestimmt werden kann. Es ist daher erfindungsgemäß eine Gasvolumenerfassungsvorrichtung vorgesehen, die das von der Gasspeicherfolie überspannte Gasvolumen in dem Biomassespeicher ermittelt. Die Gasvolumenerfassungsvorrichtung kann sich dabei verschiedener physikalischer Messprinzipien bedienen und die Form der Gasspeicherfolie beispielsweise taktil über die Gasspeicherfolie berührende Elemente, optisch über Kameras oder Sensoren oder Ähnlichen Prinzipien ermitteln. Als problematisch hat sich dabei herausgestellt, dass sich die Gasspeicherfolie in bestimmten Betriebszuständen faltet, wodurch eine nicht reproduzierbare Form der Gasspeicherfolie entsteht. Daher wird die Gasvolumenerfassungsvorrichtung, welche das Gasvolumen über die aktuelle Form der Gasspeicherfolie bestimmt, durch ein Falten der Gasspeicherfolie in ihrer Genauigkeit massiv beeinflusst. Erfindungsgemäß wird daher mindestens ein Versteifungselement für die Gasspeicherfolie vorgesehen, um eine kontinuierlichere und reproduzierbare Änderung der Form der Gasspeicherfolie abhängig von der enthaltenen Gasmenge sicherzustellen und somit eine genaue Bestimmung des Gasvolumens durch eine Gasvolumenerfassungsvorrichtung zu ermöglichen. Die erfindungsgemäße Kombination aus Gasvolumenerfassungsvorrichtung und mindestens einem Versteifungselement an der Gasspeicherfolie stellt eine einfach aufgebaute und zuverlässige Möglichkeit zur genauen und kontinuierlichen Bestimmung der im Biomassespeicher befindlichen Gasmenge zur Verfügung. Dadurch kann die durch die Vergärung erzeugte Gasmenge ständig zuverlässig bestimmt und auch überwacht werden. Der Zeitpunkt, wann eine für einen schadensfreien Betrieb der Anlage kritische Gasmenge erreicht ist, ist dadurch einfach zu bestimmen, was wiederum Beschädigungen und damit verbundene längere Ausfälle des Biomassespeichers verhindert.

Insbesondere ist das mindestens eine Versteifungselement flexibel ausgelegt. Das bedeutet, dass die Versteifungselemente zum Einhalten einer Mindestkrümmung der Gasspeicherfolie dienen. Damit das mindestens eine Versteifungselement immer optimal an der Gasspeicherfolie anliegt, ist das Versteifungselement vorzugsweise als elastisches Band oder Schnur, insbesondere als Gummiband ausgebildet. In der Ausführungsform als elastisches Band oder Schnur weist das Versteifungselement eine Vorspannung auf. Vorzugsweise ist auch die Gasspeicherfolie dehnbar.

Vorzugsweise ist das Versteifungselement an gegenüberliegenden Seiten des Behälterelementes befestigt und insbesondere diametral über das Behälterelement gespannt oder befestigt. Zur Sicherstellung einer größtmöglichen Stabilität und sicheren Verbindung ist das Versteifungselement vorzugsweise am Behälterelement, insbesondere am Rand des Behälterelementes, befestigt. In einer Weiterentwicklung dieser Ausführungsform ist das Versteifungselement in dem dem Behälterelement zugewandten Bereich der Gasspeicherfolie an dieser endseitig befestigt. Zur besseren Verbindung zwischen Versteifungselement und Gasspeicherfolie weist die Gasspeicherfolie Führungsmittel für das Versteifungselement auf. Insbesondere sind als Führungsmittel ein auf der Gasspeicherfolie vorgesehener Führungskanal, Führungsschlauch oder Führungslaschen vorgesehen.

Vorzugsweise umfasst der Biomassespeicher wenigstens eine Bogenlängenmessvorrichtung zum Erfassen einer Bogenlänge einer Messlinie zwischen zwei vorgegebenen Randpunkten an dem Behälterelement auf einer von der Gasspeicherfolie überspannten Oberfläche und zum Ermitteln des Gasvolumens unter der Gasspeicherfolie. Insbesondere umfasst die Bogenlängenmessvorrichtung eine Messschnur, die an ihrem ersten Ende an dem ersten Randpunkt des Behälterelements befestigt ist und an ihrem zweiten Ende mit einem Gewicht verbunden ist, wobei die Messschnur beweglich über den zweiten Randpunkt des flexiblen Abdeckelements geführt wird. Zur Darstellung des Gasvolumens als Messgröße ist bei dieser Ausführungsform vorzugsweise eine Skala vorgesehen, so dass eine Höhenveränderung des Gewichts ablesbar ist.

Des Weiteren umfasst die Erfindung auch die Verwendung des Biomassespeichers für den Zweck der Speicherung und Vergärung von Biomasse.

In der Zeichnung ist die Erfindung insbesondere in einem Ausführungsbeispiel schematisch dargestellt. Es zeigen:
- Fig. 1: eine Ausführungsform des erfindungsgemäßen Biomassespeichers im Querschnitt;
- Fig. 2: eine Ausführungsform des Biomassespeichers mit mehreren Versteifungselementen in Draufsicht.

In den Figuren sind gleiche oder einander entsprechende Elemente jeweils mit den gleichen Bezugszeichen bezeichnet und werden daher, sofern nicht zweckmäßig, nicht erneut beschrieben. Die in der gesamten Beschreibung enthaltenen Offenbarungen ist sinngemäß auf gleiche Teile mit gleichen Bezugszeichen bzw. gleichen Bauteilbezeichnungen übertragbar. Auch sind die in der Beschreibung gewählten Lageangaben, wie z.B. oben, unten, seitlich usw. auf die unmittelbar beschriebene sowie dargestellte Figur bezogen und sind bei einer Lageänderung sinngemäß auf die neue Lage zu übertragen. Weiterhin können auch Einzelmerkmale oder Merkmalskombinationen aus den gezeigten und beschriebenen unterschiedlichen Ausführungsbeispielen für sich eigenständige, erfinderische oder erfindungsgemäße Lösungen darstellen.

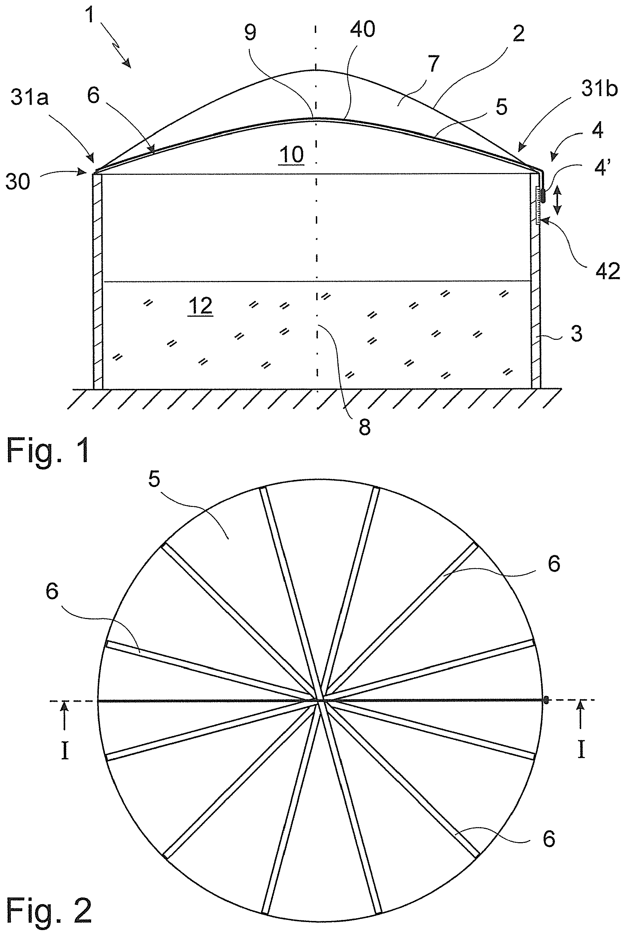

Fig. 1 zeigt einen Biomassespeicher 1 zum Speichern und Vergären von Biomasse. Der Biomassespeicher 1 setzt sich in der dargestellten Ausführungsform zusammen aus einem im Wesentlichen starren Behälterelement 3 und einer Abdeckhaube 2, die das Behälterelement 3 nach oben abschließt. Um das Gasvolumen unter der Abdeckhaube 2 zu erfassen, ist eine Gasvolumenerfassungsvorrichtung 4 vorgesehen, die das Gasvolumen gut sichtbar anzeigt. Die eigentliche Anzeigevorrichtung umfasst ein Gewicht 4', das an einer weiter unten beschriebenen Messschnur vor einer (nicht dargestellten) Skala außen an dem Behälterelement 3 hängt.

Abgedeckt wird das Behälterelement 3 durch eine Gasspeicherfolie 5. Die Gasspeicherfolie 5 ist an einem Rand 30 des Behälterelements befestigt, so dass der Innenraum des Behälterelements 3 im Wesentlichen luftdicht abgeschlossen ist.

Verstärkt wird die Gasspeicherfolie 5 durch ein Versteifungselement 6. Die Gasspeicherfolie 5 und das Versteifungselement 6 sind in Fig. 1 durch eine Doppellinie dargestellt. Mit dem Versteifungselement wird erreicht, dass die Gasspeicherfolie 5 nicht mehr unkontrolliert Falten bilden kann. Falten verändern die Ausdehnung der überspannten Oberfläche, so dass es bezüglich des eingeschlossenen Gasvolumens zu verfälschten Messergebnissen kommt. Mit anderen Worten, die Versteifungselemente stellen eine Mindestkrümmung der Gasspeicherfolie sicher. Dementsprechend kann das Versteifungselement 6 in seiner Beschaffenheit entsprechend ausgelegt sein, was im Folgenden an einigen Beispielen erläutert wird, wobei die Aufstellung der Beispiele selbstverständlich nicht erschöpfend ist.

Vorzugsweise ist das Versteifungselement 6 flexibel ausgelegt. Damit das Versteifungselement 6 optimal seinen Zweck erfüllt, werden darüber hinaus vorzugsweise mehrere Versteifungselemente 6 eingesetzt, die idealerweise jeweils an gegenüberliegenden Seiten des Behälterelementes 3 angebracht sind. Dies ist in Fig. 2 dargestellt, in der ein Biomassespeicher in Draufsicht gezeigt ist. Die sechs gezeigten Versteifungselemente 6 sind bei dieser Ausführungsform diametral über das Behälterelement 3 gespannt und an dessen Rand 30 befestigt. Auf diese Art ergibt sich eine Unterstützung der Gasspeicherfolie 5, die für eine geringe Faltenbildung ausreichend dicht ist.

Da die Versteifungselemente 6 gegen den Druck in dem Gasvolumen 10 unter der Gasspeicherfolie 5 arbeiten, sind die Versteifungselemente 6 vorzugsweise als elastische Bänder oder Schnüre, d.h. insbesondere als Gummiband ausgebildet. Die Ausbildung als Gummiband bedeutet implizit, dass das Versteifungselement 6 eine Vorspannung aufweist. Befestigt wird das Versteifungselement 6 am Behälterelement 3, insbesondere am Rand 30 des Behälterelementes 3.

Ebenso, wie die Beschaffenheit des Versteifungselements 6 je nach Anwendungsfall unterschiedlich sein kann, wird auch die Verbindung zwischen dem Versteifungselement 6 und der Gasspeicherfolie 5 je nach Anwendungsfall unterschiedlich gewählt werden. Insbesondere weist die Gasspeicherfolie 5 Führungsmittel für das Versteifungselement 6 auf. Die Führungsmittel können dabei in Form eines auf der Gasspeicherfolie 5 vorgesehenen Führungskanals, eines Führungsschlauchs oder in Form von Führungslaschen vorgesehen sein. Je nach Anwendungsfall wird das Versteifungselement 6 auf der Innenseite oder der Außenseite der Gasspeicherfolie 5 geführt und befestigt. Insbesondere ist das Versteifungselement 6 in dem dem Behälterelement 3 zugewandten Bereich der Gasspeicherfolie 5 an dieser endseitig befestigt.

In dem Behälterelement 3 eingelagerte Biomasse 12 vergärt mit der Zeit, und dabei werden Gase freigesetzt, die sich unter der Gasspeicherfolie 5 sammeln. Die Gasspeicherfolie 5 ist vorzugsweise dehnbar ausgelegt. Das von der Gasspeicherfolie 5 überspannte Gasvolumen in dem Biomassespeicher ist in Fig. 1 mit der Ziffer 10 bezeichnet.

Um das Gasvolumen 10 unter der Gasspeicherfolie 5 quantitativ zu erfassen, ist eine Bogenlängenmessvorrichtung vorgesehen. Die Bogenlängenmessvorrichtung umfasst eine Messschnur 40, die an dem Rand 30 des Behälterelements 3 befestigt ist. Genauer ist die Messschnur 40 mit ihrem ersten Ende an einem ersten Randpunkt 31a an dem Behälterelement 3 befestigt. Von dort aus verläuft sie auf der Oberfläche der Gasspeicherfolie 5 zu einem zweiten Randpunkt 31b an dem Behälterelement 3 und über diesen hinaus. Die Messschnur 40 wird an dem zweiten Randpunkt 31b beweglich geführt. Mit ihrem zweiten Ende ist die Messschnur 40 mit einem Gewicht 4' verbunden, das frei an der Außenwand des Behälterelements 3 hängt. Insbesondere ist in Höhe des Gewichts 4' eine Skala 42 angeordnet, die die Höhe des Gewichts über dem Boden anzeigt und damit die "verbrauchte" Bogenlänge der Messschnur über der Gasspeicherfolie 5. Mit dieser Bogenlängenmessvorrichtung lässt sich die Bogenlänge einer Messlinie zwischen zwei vorgegebenen Randpunkten an dem Behälterelement auf einer von der Gasspeicherfolie 5 überspannten Oberfläche erfassen und somit eine Aussage über das von der Gasspeicherfolie umschlossene Gasvolumen machen. Die Erfindung ist nicht auf eine Messschnur 40 und ein Gewicht 4' beschränkt, sondern es können mehrere Bogenlängenvorrichtungen eingesetzt werden, um verschiedene Bogenlängen zu vergleichen. Bei gleichem Abstand der Randpunkte 31a, 31b für jede Messschnur 40 stimmen die angezeigten Gewichtshöhen auf den Skalen 42 für alle Messschnüre überein. Es versteht sich für den Fachmann, dass sich die Randpunkte 31a, 31b für die Messschnur oder für jede der mehreren Messschnüre auf dem Rand des Behälterelements 3 vorzugsweise diametral gegenüberliegen.

Die jetzt mit der Anmeldung und später eingereichten Ansprüche sind ohne Präjudiz für die Erzielung weitergehenden Schutzes. Sollte sich hier bei näherer Prüfung, insbesondere auch des einschlägigen Standes der Technik, ergeben, dass das eine oder andere Merkmal für das Ziel der Erfindung zwar günstig, nicht aber entscheidend wichtig ist, so wird selbstverständlich schon jetzt eine Formulierung angestrebt, die ein solches Merkmal, insbesondere im Hauptanspruch, nicht mehr aufweist. Auch eine solche Unterkombination ist von der Offenbarung dieser Anmeldung abgedeckt.

Es ist weiter zu beachten, dass die in den verschiedenen Ausführungsformen beschriebenen und in den Figuren gezeigten Ausgestaltungen und Varianten der Erfindung beliebig untereinander kombinierbar sind. Dabei sind einzelne oder mehrere Merkmale beliebig gegeneinander austauschbar. Diese Merkmalskombinationen sind ebenso mit offenbart.

Die in den abhängigen Ansprüchen angeführten Rückbeziehungen weisen auf die weitere Ausbildung des Gegenstandes des Hauptanspruches durch die Merkmale des jeweiligen Unteranspruches hin. Jedoch sind diese nicht als ein Verzicht auf die Erzielung eines selbständigen, gegenständlichen Schutzes für die Merkmale der rückbezogenen Unteransprüche zu verstehen.

Merkmale, die nur in der Beschreibung offenbart wurden oder auch Einzelmerkmale aus Ansprüchen, die eine Mehrzahl von Merkmalen umfassen, können jederzeit als von erfindungswesentlicher Bedeutung zur Abgrenzung vom Stande der Technik in den oder die unabhängigen Anspruch/Ansprüche übernommen werden, und zwar auch dann, wenn solche Merkmale im Zusammenhang mit anderen Merkmalen erwähnt wurden beziehungsweise im Zusammenhang mit anderen Merkmalen besonders günstige Ergebnisse erreichen.

## Patentansprüche

1. Biomassespeicher zum Vergären von Biomasse, der umfasst:
ein im Wesentlichen starres Behälterelement (3) zur Aufnahme der Biomasse (12) und
eine Abdeckhaube (2) und
eine unterhalb der Abdeckhaube (2) angeordnete Gasspeicherfolie (5) zum Verschließen des Behälterelements (3), die an einem Rand (30) des Behälterelements (3) befestigt ist, so dass der Biomassespeicher (1) im Wesentlichen luftdicht abgeschlossen ist und
eine Gasvolumenerfassungsvorrichtung (4) zum Ermitteln und Anzeigen eines von der Gasspeicherfolie (5) überspannten Gasvolumens (10) in dem Biomassespeicher (1), wobei an der Gasspeicherfolie (5) mindestens ein Versteifungselement (6) angeordnet ist.

2. Biomassespeicher nach Anspruch 1, **dadurch gekennzeichnet, dass** das Versteifungselement (6) flexibel ist.

3. Biomassespeicher nach einem der vorhergehenden Ansprüche, **dadurch gekennzeichnet, dass** das Versteifungselement (6) an gegenüberliegenden Seiten des Behälterelementes (3), bevorzugt diametral über das Behälterelement (3) gespannt oder befestigt ist.

4. Biomassespeicher nach einem der vorhergehenden Ansprüche, **dadurch gekennzeichnet, dass** das Versteifungselement (6) als elastische Band oder Schnur, insbesondere als Gummiband ausgebildet ist.

5. Biomassespeicher nach einem der vorhergehenden Ansprüche, **dadurch gekennzeichnet, dass** das als elastisches Band oder Schnur ausgebildete Versteifungselement (6) eine Vorspannung aufweist.

6. Biomassespeicher nach einem der vorhergehenden Ansprüche, **dadurch gekennzeichnet, dass** das Versteifungselement (6) am Behälterelement (3), insbesondere am Rand (30) des Behälterelementes (3), befestigt ist.

7. Biomassespeicher nach einem der vorhergehenden Ansprüche, **dadurch gekennzeichnet, dass** das Versteifungselement (6) in dem dem Behälterelement (3) zugewandten Bereich der Gasspeicherfolie (5) an dieser endseitig befestigt ist.

8. Biomassespeicher nach einem der vorhergehenden Ansprüche, **dadurch gekennzeichnet, dass** die Gasspeicherfolien (5) Führungsmittel für das Versteifungselement (6) aufweist.

9. Biomassespeicher nach einem der vorhergehenden Ansprüche, **dadurch gekennzeichnet, dass** als Führungsmittel ein auf der Gasspeicherfolie (5) vorgesehener Führungskanal, Führungsschlauch oder Führungslaschen vorgesehen sind.

10. Biomassespeicher nach einem der vorhergehenden Ansprüche, **dadurch gekennzeichnet, dass** bei dem die Gasvolumenerfassungsvorrichtung (4) wenigstens eine Bogenlängenmessvorrichtung umfasst zum Erfassen einer Bogenlänge einer Messlinie zwischen zwei vorgegebenen Randpunkten an dem Behälterelement auf einer von der Gasspeicherfolie (5) überspannten Oberfläche.

11. Biomassespeicher nach einem der vorangehenden Ansprüche, **dadurch gekennzeichnet, dass** die Gasspeicherfolie (5) dehnbar ist.

## Claims

1. Biomass storage for the fermentation of biomass, comprising:
An essentially rigid container element (3) for holding the biomass (12), and
a cover hood (2), and
a gas-storage foil (5) positioned beneath the cover hood (2) for sealing the container element (3), the foil being attached to an edge (30) of the container element (3) so that the biomass storage (1) is essentially hermetically sealed, and
a measurement device (4) for the gas volume for determining and indicating a gas volume (10) spanned by the gas-storage foil (5) in the biomass storage (1), wherein at the gas-storage foil (5) at least one support element (6) is arranged.

2. Biomass storage according to claim 1, **characterized by** a flexible support element (6).

3. Biomass storage according to one of the preceding claims, **characterized in that** the support element (6) is spanned over or attached to the container element (3) on opposing faces of the container element (3), preferably diametrically.

4. Biomass storage according to one of the preceding claims, **characterized in that** the support element (6) is designed as elastic band or string, in particular as rubber band.

5. Biomass storage according to one of the preceding claims, **characterized in that** the support element (6) designed as elastic band or string is preloaded.

6. Biomass storage according to one of the preceding claims, **characterized in that** the support element (6) is attached to the container element (3), in particular the edge (30) of the container element (3).

7. Biomass storage according to one of the preceding claims, **characterized in that** the support element (6) is fastened in the area of the gas-storage foil (5) facing the container element (3) to the end of the gas-storage foil.

8. Biomass storage according to one of the preceding claims, **characterized in that** the gas-storage foils (5) are provided with guide means for the support element (6).

9. Biomass storage according to one of the preceding claims, **characterized in that** as guide means a guide channel, guide hose, or guide flaps provided on the gas-storage foil (5) are provided.

10. Biomass storage according to one of the preceding claims, **characterized in that** the gas volume-measuring device (4) comprises at least an arc length-measuring device for recording an arc length of a measurement line between two determined boundary points at the container element on a surface spanned-over by the gas-storage foil (5).

11. Biomass storage according to one of the preceding claims, **characterized by** an elastic gas-storage foil (5).

## Revendications

1. Accumulateur à biomasse destiné à la fermentation de biomasses comprenant :
un conteneur (3) essentiellement rigide contenant la biomasse (12),
un couvercle (2),
une bâche d'accumulateur à biomasse (5) située en-dessous du couvercle (2), fixée sur un bord (30) du conteneur (3) et servant à fermer le conteneur (3) afin de fermer de façon étanche l'accumulateur à biomasse (1)
et un dispositif de saisie du volume de gaz (4) permettant de déterminer et d'afficher le volume de gaz (10) situé en-dessous de la bâche d'accumulateur à biomasse (5) à l'intérieur de l'accumulateur à biomasse (1) avec au moins un élément raidisseur (6) qui est situé au niveau de la bâche d'accumulateur à biomasse (5).

2. Accumulateur à biomasse selon la revendication 1, **caractérisé en ce que** l'élément raidisseur (6) est flexible.

3. Accumulateur à biomasse selon une des revendications précédentes, **caractérisé en ce que** l'élément raidisseur (6) est fixé au niveau de bords opposés du conteneur (3) et de préférence étendu ou fixé diamétralement au-dessus du conteneur (3).

4. Accumulateur à biomasse selon une des revendications précédentes, **caractérisé en ce que** l'élément raidisseur (6) est formé par une sangle élastique ou par une corde et en particulier par une sangle en caoutchouc.

5. Accumulateur à biomasse selon une des revendications précédentes, **caractérisé en ce que** l'élément raidisseur (6) formé par une sangle élastique ou par une corde est précontraint.

6. Accumulateur à biomasse selon une des revendications précédentes, **caractérisé en ce que** l'élément raidisseur (6) est fixé au conteneur (3) et en particulier au niveau du bord (30) du conteneur (3).

7. Accumulateur à biomasse selon une des revendications précédentes, **caractérisé en ce que** l'élément raidisseur (6) est situé au niveau de la face de la bâche d'accumulateur à biomasse (5) tournée vers le conteneur (3) et celui-ci étant fixé à l'extrémité de celle-ci.

8. Accumulateur à biomasse selon une des revendications précédentes, **caractérisé en ce que** la bâche d'accumulateur à biomasse (5) possède des moyens de guidage pour l'élément raidisseur (6).

9. Accumulateur à biomasse selon une des revendications précédentes, **caractérisé en ce que** le moyen de guidage prévu au niveau de la bâche d'accumulateur à biogaz (5) peut être un canal de guidage, un tube de guidage ou des ergots de guidage.

10. Accumulateur à biomasse selon une des revendications précédentes, **caractérisé en ce que** le dispositif de saisie du volume de gaz (4) comprend au moins un dispositif de mesure de longueur d'arc destiné à mesurer la longueur d'arc d'une ligne de mesure entre deux points fixes situés au niveau du bord du conteneur et passant par la surface de la bâche d'accumulateur à biogaz (5).

11. Accumulateur à biomasse selon une des revendications précédentes, **caractérisé en ce que** la bâche d'accumulateur à biogaz est extensible.
